# EUROPEAN PATENT APPLICATION

(11) **EP 4 685 226 A1**
(43) Date of publication of application: **28.01.2026**
(21) Application number: 23928899.6
(22) Date of filing: 28.12.2023
(51) Int. Cl.: C12N 1/20, A61K 35/74, A61K 39/00, C12R 1/01

(54) **NOVEL PREVOTELLA MERDAE IMMUNOACTIS STRAIN AND USE THEREOF**

(30) Priority: 21.03.2023 KR 20230036857
(71) Applicant: The Asan Foundation, Seoul 05505 (KR); UNIVERSITY OF ULSAN FOUNDATION FOR INDUSTRY COOPERATION, Nam-gu Ulsan 44610 (KR); Gwangju Institute of Science and Technology, Gwangju 61005 (KR)
(72) Inventor: PARK, Sook Ryun, Seoul 05649 (KR); KWEON, Mi-Na, Yangpyeong-gun, Gyeonggi-do 12503 (KR); PARK, Hansoo, Seoul 06356 (KR); KIM, Gihyeon, Seongnam-si, Gyeonggi-do 13357 (KR)
(74) Representative: Dehns
(86) International application number: PCT/KR2023/021918
(87) International publication number: WO 2024/195976

(57) **Abstract**

The present invention relates to a novel *Prevotella merdae* Immunoactis strain, use thereof, and the like. The inventors of the present invention have confirmed that the novel *Prevotella merdae* Immunoactis strain (accession number: KCTC 14922BP) increases the proliferation of T cells, increases IFN-γ secretion from T cells, increases the expression or activity level of IFN-γ or Cxcl9, reduces the expression or activity level of Ccl22, and enhances the anticancer efficacy of immune checkpoint inhibitors. Therefore, the novel *Prevotella merdae* Immunoactis strain or a culture broth thereof, according to the present invention, is expected to be effectively used for enhancing immunity and increasing the anticancer effect of immune checkpoint inhibitors.

## Description

### [Technical Field]

The present invention relates to a novel *Prevotella merdae* Immunoactis strain and use thereof.

This application claims priority to and the benefit of Korean Patent Application No. 10-2023-0036857, filed on March 21, 2023, the disclosure of which is incorporated herein by reference in its entirety.

### [Background Art]

Cancer is one of the intractable diseases that humankind must solve, and huge amounts of capital are being invested in the development of a cure for it worldwide. In Korea, it has been the top leading cause of death since the 1990s, and more than 100,000 people are diagnosed with cancer each year, and more than 60,000 people die due to cancer. The causes of cancer include smoking, ultraviolet rays, chemicals, food, stress, and other environmental factors, but since the causes are diverse, it is not only difficult to develop treatments, but the effectiveness of treatments also varies according to the site of occurrence.

Cancer treatment includes surgical removal of tumor tissue, radiation therapy, chemotherapy, and immunotherapy. Different anticancer drugs are applied in cancer treatment according to each cancer type. In general, chemical anticancer drugs prescribed to cancer patients affect the survival mechanism of not only cancer cells but also normal cells, causing side effects such as hair loss, fever, diarrhea, rashes, and decreased immunity in patients. Based on genetic research on cancer, targeted anticancer drugs that suppress specific features of cancer's characteristic genetic mutations were developed, and thus the side effects caused by existing chemical anticancer drugs have been greatly reduced. However, there is a problem that a 100% continuous anticancer effect of targeted anticancer drugs cannot be expected because cancer cells, which mutate very quickly, induce anticancer drug resistance. Recently, research on the tumor microenvironment has been actively conducted, and cancer immunotherapies targeting various immune checkpoints that control the patient's immunity have been developed and are being used as treatments for patients. Among them, cancer immunotherapies that suppress programmed death-1 (PD-1)/ programmed death-ligand 1 (PD-L1) are known to exhibit high therapeutic responses in patients with melanoma, stomach cancer, and lung cancer. These cancer immunotherapies have the function of suppressing the proliferation of cancer cells by activating the function of immune cells in the tumor microenvironment. However, cancer immunotherapies also do not exhibit the same anticancer effect in all patients.

### [Disclosure]

### [Technical Problem]

The technical problem to be solved by the present invention is to provide a novel *Prevotella merdae* Immunoactis strain, a composition for enhancing immunity comprising the strain or a culture solution thereof as an active ingredient, and a composition for increasing an anticancer effect, and the inventors of the present invention confirmed that the novel *Prevotella merdae* Immunoactis strain increases T cell proliferation, increases IFN-γ secretion from T cells, and enhances the anticancer efficacy of immune checkpoint inhibitors.

Accordingly, an object of the present invention is to provide a *Prevotella merdae* Immunoactis strain deposited under accession number KCTC 14922BP.

Another object of the present invention is to provide a composition for enhancing immunity comprising the *Prevotella merdae* Immunoactis strain of the present invention or a culture solution thereof as an active ingredient.

Still another object of the present invention is to provide a pharmaceutical composition for increasing an anticancer effect comprising the *Prevotella merdae* Immunoactis strain of the present invention or a culture solution thereof as an active ingredient.

Yet another object of the present invention is to provide a food composition for increasing an anticancer effect comprising the *Prevotella merdae* Immunoactis strain of the present invention or a culture solution thereof as an active ingredient.

Yet another object of the present invention is to provide a pharmaceutical composition for preventing or treating cancer comprising the *Prevotella merdae* Immunoactis strain of the present invention or a culture solution thereof; and an immune checkpoint inhibitor as active ingredients.

However, technical problems to be solved in the present invention are not limited to the above-described problems, and other problems which are not described herein will be fully understood by those of ordinary skill in the art from the following descriptions.

### [Technical Solution]

To achieve the above object of the present invention, the present invention provides a *Prevotella merdae* Immunoactis strain deposited under accession number KCTC 14922BP.

Additionally, the present invention provides a composition for enhancing immunity comprising the *Prevotella merdae* Immunoactis strain of the present invention or a culture solution thereof as an active ingredient.

Additionally, the present invention provides a pharmaceutical composition for increasing an anticancer effect comprising the *Prevotella merdae* Immunoactis strain of the present invention or a culture solution thereof as an active ingredient.

Additionally, the present invention provides a food composition for increasing an anticancer effect comprising the *Prevotella merdae* Immunoactis strain of the present invention or a culture solution thereof as an active ingredient.

Additionally, the present invention provides a pharmaceutical composition for preventing or treating cancer comprising the *Prevotella merdae* Immunoactis strain of the present invention or a culture solution thereof; and an immune checkpoint inhibitor as active ingredients.

In one embodiment of the present invention, the strain may comprise a DNA sequence encoding a 16S rRNA sequence of SEQ ID NO: 1, but is not limited thereto.

In another embodiment of the present invention, the strain may enhance immunity or increase the anticancer effect of an anticancer drug, but is not limited thereto.

In yet another embodiment of the present invention, the pharmaceutical composition for increasing the anticancer effect may be used in combination with an anticancer drug, but is not limited thereto.

In yet another embodiment of the present invention, the immune checkpoint inhibitor may be a PD-L1 inhibitor or a PD-1 inhibitor, but is not limited thereto.

In yet another embodiment of the present invention, the PD-L1 inhibitor may be one or more selected from the group consisting of atezolizumab, avelumab, durvalumab, envafolimab, cosibelimab, AUNP12, CA-170, and BMS-986189, but is not limited thereto.

In yet another embodiment of the present invention, the PD-1 inhibitor may be one or more selected from the group consisting of nivolumab, pembrolizumab, cemiplimab, spartalizumab, camrelizumab, sintilimab, tislelizumab, toripalimab, dostarlimab, INCMGA00012, AMP-224, and AMP-514, but is not limited thereto.

In yet another embodiment of the present invention, the *Prevotella merdae* Immunoactis strain or a culture solution thereof; and the immune checkpoint inhibitor may be administered simultaneously or sequentially, but is not limited thereto.

In yet another embodiment of the present invention, the *Prevotella merdae* Immunoactis strain or a culture solution thereof may satisfy one or more of the following characteristics, but is not limited thereto:
(a) increasing the proliferation of CD4⁺ T cells and CD8⁺ T cells;
(b) increasing the secretion of interferon-γ (IFN-γ) by T cells;
(c) increasing the expression or activity level of Inf-γ or Cxcl9; and
(b) decreasing the expression or activity level of Cc122.

In yet another embodiment of the present invention, the cancer may be one or more selected from the group consisting of colorectal cancer, breast cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney cancer, ureteral cancer, renal cell carcinoma, renal pelvic carcinoma, central nervous system tumors, primary central nervous system lymphoma, spinal cord tumors, brainstem glioma, and pituitary adenoma, but is not limited thereto.

Additionally, the present invention provides a method of enhancing immunity, comprising administering a composition comprising the *Prevotella merdae* Immunoactis strain of the present invention or a culture solution thereof to a subject in need thereof.

Additionally, the present invention provides a use of a composition comprising the *Prevotella merdae* Immunoactis strain of the present invention or a culture solution thereof for enhancing immunity.

Additionally, the present invention provides a use of the *Prevotella merdae* Immunoactis strain of the present invention or a culture solution thereof for preparing an immunity-enhancing agent.

Additionally, the present invention provides a method of enhancing the anticancer effect of an anticancer drug, comprising administering a composition comprising the *Prevotella merdae* Immunoactis strain of the present invention or a culture solution thereof to a subject in need thereof.

Additionally, the present invention provides a use of a composition comprising the *Prevotella merdae* Immunoactis strain of the present invention or a culture solution thereof for increasing the anticancer effect of an anticancer drug.

Additionally, the present invention provides a use of the *Prevotella merdae* Immunoactis strain of the present invention or a culture solution thereof for preparing an agent for increasing the anticancer effect of an anticancer drug.

Additionally, the present invention provides a method of preventing or treating cancer, comprising administering a composition comprising the *Prevotella merdae* Immunoactis strain of the present invention or a culture solution thereof; and an immune checkpoint inhibitor to a subject in need thereof.

Additionally, the present invention provides a use of a composition comprising the *Prevotella merdae* Immunoactis strain of the present invention or a culture solution thereof; and an immune checkpoint inhibitor for preventing or treating cancer.

Additionally, the present invention provides a use of a composition comprising the *Prevotella merdae* Immunoactis strain of the present invention or a culture solution thereof; and an immune checkpoint inhibitor for preparing a cancer treatment agent.

### [Advantageous Effects]

The inventors of the present invention have confirmed that the novel *Prevotella merdae* Immunoactis strain (accession number: KCTC 14922BP) increases the proliferation of T cells, increases IFN-γ secretion from T cells, increases the cancer cell killing ability of T cells, increases the expression or activity level of IFN-γ or Cxcl9 in the tumor microenvironment, and decreases the expression or activity level of Ccl22, thereby enhancing the anticancer efficacy of immune checkpoint inhibitors. Therefore, the novel *Prevotella merdae* Immunoactis strain or a culture solution thereof according to the present invention is expected to be effectively used for enhancing immunity and increasing the anticancer effect of immune checkpoint inhibitors.

### [Description of Drawings]

FIG. 1 shows the proliferation effect of CD4⁺ T cells when treated with the culture supernatant of the *Prevotella merdae* Immunoactis strain.
FIG. 2 shows the proliferation effect of CD8⁺ T cells when treated with the culture supernatant of the *Prevotella merdae* Immunoactis strain.
FIG. 3 shows IFN-γ secreted from CD4⁺ T cells and CD8⁺ T cells treated with the culture supernatant of the *Prevotella merdae* Immunoactis strain.
FIG. 4 shows that the combination of the *Prevotella merdae* Immunoactis strain and α-PD-1 antibody reduces the growth of colon cancer tumors.
FIG. 5 shows the effect of the combination of the *Prevotella merdae* Immunoactis strain and α-PD-1 antibody on the expression of Inf-γ, Cxcl9, and Ccl22.
FIG. 6 shows that the combination of the *Prevotella merdae* Immunoactis strain and α-PD-1 antibody increases the populations of CD4⁺ T cells, CD4⁺CD44⁺CD62L⁻ T cells (CD4⁺ effector T cells), and TNF-α⁺ cells.
FIG. 7 shows that the combination of the *Prevotella merdae* Immunoactis strain and α-PD-1 antibody increases the populations of CD8⁺ T cells, CD8⁺CD44⁺CD62L⁻ T cells, and PD-1⁺CD8⁺ T cells.
FIG. 8 shows the results of evaluating the cytotoxicity of the *Prevotella merdae* Immunoactis strain.

### [Best Mode]

In one example of the present invention, a strain isolated from a patient who responded to anti-PD-1 treatment was identified as a novel strain belonging to the same species as the reference strain *Prevotella merdae* sp. Marseille-P4119 but being a different strain, and was named *Prevotella merdae* Immunoactis (see Example 1).

In another example of the present invention, it was confirmed that treatment with the culture supernatant of the *Prevotella merdae* Immunoactis strain significantly promoted the proliferation of CD4⁺ T cells and CD8⁺ T cells and significantly increased IFN-γ secretion from T cells (see Example 3).

In yet another example of the present invention, it was confirmed that the combined administration of the *Prevotella merdae* Immunoactis strain and a PD-1 inhibitor exhibited a significantly superior tumor growth inhibition effect (see Example 4).

In yet another example of the present invention, it was confirmed that the combined administration of the *Prevotella merdae* Immunoactis strain and a PD-1 inhibitor increased the expression of Inf-γ and Cxcl9 and decreased the expression of Ccl22 in the tumor tissue of mice. In addition, it was confirmed that the combined administration increased the populations of CD4⁺ T cells, CD4⁺CD44⁺CD62L⁻ T cells (CD4⁺ effector T cells), TNF-α⁺ cells, CD8⁺ T cells, CD8⁺CD44⁺CD62L⁻ T cells, and PD-1⁺CD8⁺ T cells (see Example 5).

In yet another example of the present invention, it was confirmed that when OT-1 T cells were treated with the culture supernatant of the *Prevotella merdae* Immunoactis strain, the ovalbumin-pulsed T cells treated with the culture supernatant of the *Prevotella merdae* Immunoactis strain killed MC38 colon cancer cells more effectively.

Therefore, the *Prevotella merdae* Immunoactis strain or a culture solution thereof according to the present invention is expected to be usefully employed not only for enhancing immunity and increasing anticancer effect, but also as an effective combination anticancer composition with an immune checkpoint inhibitor based on a synergistic effect with the immune checkpoint inhibitor.

Hereinafter, the present invention will be described in detail.

The present invention provides a *Prevotella merdae* Immunoactis strain deposited under accession number KCTC 14922BP. According to one example of the present invention, analysis of the whole genome and 16S rRNA sequence for the identification of the strain confirmed that the 16S rRNA of the strain was encoded with the nucleotide sequence of SEQ ID NO: 1.

According to one example of the present invention, the strain was identified as a novel strain belonging to the same species as the reference strain *Prevotella merdae* sp. Marseille-P4119, with a similarity of 97.82%, but being a different strain.

Accordingly, the inventors of the present invention named the strain *Prevotella merdae* Immunoactis and deposited it with the Korean Collection for Type Cultures (KCTC) of the Korea Research Institute of Bioscience and Biotechnology on March 24, 2022, under accession number KCTC 14922BP.

According to one example of the present invention, the strain increases T cell proliferation, increases IFN-γ secretion from T cells, increases the expression or activity level of IFN-γ or Cxcl9, and decreases the expression or activity level of Ccl22, and can also enhance the anticancer efficacy of immune checkpoint inhibitors, thereby being characterized by enhancing immunity or increasing anticancer effect.

Accordingly, the present invention provides a composition for enhancing immunity, a pharmaceutical composition for increasing anticancer effect, or a pharmaceutical composition for preventing or treating cancer, comprising the *Prevotella merdae* Immunoactis strain of the present invention or a culture solution thereof as an active ingredient.

Additionally, the present invention provides a pharmaceutical composition for preventing or treating cancer comprising the *Prevotella merdae* Immunoactis strain of the present invention or a culture solution thereof; and an immune checkpoint inhibitor as active ingredients.

In the present invention, "enhancing immunity" refers to increasing the immune response or activity of the immune system in the body, and immunity is broadly classified into innate immunity and acquired immunity. Innate immunity functions to protect the body against antigen invasion by responding nonspecifically without specific immunological memory against the antigen. Innate immunity includes various anatomical and biological defense mechanisms such as the skin, mucosal tissues, strongly acidic gastric acid, and complements present in blood, and the cells responsible for innate immunity include phagocytic macrophages, polymorphonuclear leukocytes, and natural killer (NK) cells that kill infected cells and cancer cells. Acquired immunity refers to a stronger immune response including immunological memory that remembers antigen markers, is antigen-specific, and requires the recognition of non-self antigens through the antigen presentation process.

The *Prevotella merdae* Immunoactis strain of the present invention promotes the proliferation of T cells and has an excellent effect of promoting IFN-γ secretion from T cells, thereby enhancing both innate and acquired immune functions.

In the present invention, "increasing anticancer effect" may refer to, for example, enhancing the tumor growth inhibitory effect of an anticancer drug when used in combination with the anticancer drug, but is not limited thereto. In one example of the present invention, it was confirmed that the combined administration of an anti-PD-1 antibody, which is an immune checkpoint inhibitor, and the culture supernatant of the *Prevotella merdae* Immunoactis strain not only suppressed the growth of colon cancer but also significantly increased the proportion of T cells (see Example 3).

Accordingly, in the present invention, the pharmaceutical composition for increasing anticancer effect may be used in combination with an anticancer drug, but is not limited thereto.

In the present invention, the anticancer drug may be one or more selected from the group consisting of cisplatin, 5-fluorouracil, paclitaxel, doxorubicin, daunorubicin, vinblastine, vincristine, actinomycin D, teniposide, etoposide, cyclophosphamide, epirubicin, adriamycin, daunomycin, an immune checkpoint inhibitor, and mitomycin-C.

As used herein, the term "culturing" refers to all acts performed to grow microorganisms under artificially controlled environmental conditions, and includes "fermentation" in the context of the present invention.

In the present invention, the cell mass of the *Prevotella merdae* Immunoactis may include not only the live bacteria obtained from a culture medium, but also any processed forms of lactic acid bacteria known to those skilled in the art, such as cell lysates, dried forms, and frozen forms.

As used herein, the term "culture solution" includes "fermentation broth" and may include the culture solution itself obtained by culturing in a liquid medium, and processed forms derived from the culture solution, such as a filtrate or supernatant obtained by filtering or centrifuging the culture solution to remove the strain, but is not limited thereto.

In the present invention, the immune checkpoint inhibitor may be a PD-L1 inhibitor or a PD-1 inhibitor, but is not limited thereto.

In the present invention, the PD-L1 inhibitor may be an anti-PD-L1 antibody, but is not limited thereto. The PD-L1 inhibitor may be one or more selected from the group consisting of atezolizumab, avelumab, durvalumab, envafolimab, cosibelimab, AUNP12, CA-170, and BMS-986189, but is not limited thereto.

In the present invention, the PD-1 inhibitor may be an anti-PD-1 antibody, but is not limited thereto. The PD-1 inhibitor may be one or more selected from the group consisting of nivolumab, pembrolizumab, cemiplimab, spartalizumab, camrelizumab, sintilimab, tislelizumab, toripalimab, dostarlimab, INCMGA00012, AMP-224, and AMP-514, but is not limited thereto.

In the present invention, the *Prevotella merdae* Immunoactis strain or a culture solution thereof; and the immune checkpoint inhibitor may be mixed at a volume ratio of 0.5 to 10:1, 0.5 to 9:1, 0.5 to 8:1, 0.5 to 7:1, 0.5 to 6:1, 0.5 to 5:1, 0.5 to 4.5:1, 1 to 10:1, 1 to 9:1, 1 to 8:1, 1 to 7:1, 1 to 6:1, 1 to 5:1, 1 to 4.5:1, 2 to 10:1, 2 to 9:1, 2 to 8:1, 2 to 7:1, 2 to 6:1, 2 to 5:1, 2 to 4.5:1, 3 to 10:1, 3 to 9:1, 3 to 8:1, 3 to 7:1, 3 to 6:1, 3 to 5:1, 3 to 4.5:1, 3.5 to 4.5:1, or 4:1, but is not limited thereto. In addition, the *Prevotella merdae* Immunoactis strain or a culture solution thereof and the immune checkpoint inhibitor may be separately administered in the above volume ratio.

In addition, the *Prevotella merdae* Immunoactis strain or a culture solution thereof and the PD-1 inhibitor may be mixed at a volume ratio of 0.1 to 10:1, 0.1 to 9:1, 0.1 to 8:1, 0.1 to 7:1, 0.1 to 6:1, or 0.1 to 5:1, but is not limited thereto.

In the present invention, the *Prevotella merdae* Immunoactis strain or a culture solution thereof may be administered simultaneously with or sequentially to the immune checkpoint inhibitor, but is not limited thereto. In the present invention, the composition comprising the *Prevotella merdae* Immunoactis strain or a culture solution thereof as an active ingredient may be formulated to be present in a single container as a mixture with the immune checkpoint inhibitor, or may be formulated to be present in separate containers for simultaneous or sequential administration. Additionally, the composition comprising the *Prevotella merdae* Immunoactis strain or a culture solution thereof as an active ingredient may be administered prior to the treatment with the immune checkpoint inhibitor, or may be administered as a second-line therapy after the treatment with the immune checkpoint inhibitor, but is not limited thereto.

In the present invention, the *Prevotella merdae* Immunoactis strain or a culture solution thereof may satisfy one or more of the following characteristics, but is not limited thereto:
(a) increasing the proliferation of CD4⁺ T cells and CD8⁺ T cells;
(b) increasing the secretion of interferon-γ (IFN-γ) by T cells;
(c) increasing the expression or activity level of Inf-γ or Cxcl9; and
(b) decreasing the expression or activity level of Ccl22.

As used in the present invention, the term "cancer" refers to a disease related to the regulation of cell death, which results from the excessive proliferation of cells when the balance of normal apoptosis is disrupted. These abnormally proliferating cells may, in some cases, invade surrounding tissues and organs to form a mass and cause the destruction or distortion of normal bodily structures, a condition generally referred to as a tumor. Tumors are generally classified into benign tumors and malignant tumors. Malignant tumors grow much faster than benign tumors, invade surrounding tissues, and metastasize, thereby posing a life-threatening risk. Such malignant tumors are commonly referred to as "cancer."

In the present invention, the cancer may be one or more selected from the group consisting of breast cancer, colorectal cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney cancer, ureteral cancer, renal cell carcinoma, renal pelvic carcinoma, central nervous system tumors, primary central nervous system lymphoma, spinal cord tumors, brainstem glioma, and pituitary adenoma, and according to one example of the present invention, the cancer may be colon cancer, but is not limited thereto.

The content of the *Prevotella merdae* Immunoactis strain or a culture solution thereof in the composition of the present invention may be appropriately adjusted depending on the symptoms of the disease, the degree of progression, the condition of the subject, and the like, and may be, for example, 0.0001 to 99.9% by weight, or 0.001 to 50% by weight, based on the total weight of the composition, but is not limited thereto. The above content ratio is based on the dried weight after removal of the solvent.

The pharmaceutical composition according to the present invention may further include a suitable carrier, excipient, and diluent which are commonly used in the preparation of pharmaceutical compositions. The excipient may be, for example, one or more selected from the group consisting of a diluent, a binder, a disintegrant, a lubricant, an adsorbent, a humectant, a film-coating material, and a controlled release additive.

The pharmaceutical composition according to the present invention may be used by being formulated, according to commonly used methods, into a form such as powders, granules, sustained-release-type granules, enteric granules, liquids, eye drops, elixirs, emulsions, suspensions, spirits, troches, aromatic water, lemonades, tablets, sustained-release-type tablets, enteric tablets, sublingual tablets, hard capsules, soft capsules, sustained-release-type capsules, enteric capsules, pills, tinctures, soft extracts, dry extracts, fluid extracts, injections, capsules, perfusates, or a preparation for external use, such as plasters, lotions, pastes, sprays, inhalants, patches, sterile injectable solutions, or aerosols. The preparation for external use may have a formulation such as creams, gels, patches, sprays, ointments, plasters, lotions, liniments, pastes, or cataplasmas.

As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used.

For formulation, commonly used diluents or excipients such as fillers, thickeners, binders, wetting agents, disintegrants, and surfactants are used.

As additives of tablets, powders, granules, capsules, pills, and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel^{®}; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol, and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution, and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose, and sodium carboxymethylcellulose may be used.

In syrups according to the present invention, a white sugar solution, other sugars or sweeteners, and the like may be used, and as necessary, a fragrance, a colorant, a preservative, a stabilizer, a suspending agent, an emulsifier, a viscous agent, or the like may be used.

In emulsions according to the present invention, purified water may be used, and as necessary, an emulsifier, a preservative, a stabilizer, a fragrance, or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC), HPMC 1828, HPMC 2906, HPMC 2910, and the like may be used, and as necessary, a surfactant, a preservative, a stabilizer, a colorant, and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate, and benzene benzoate; cosolvents such as sodium benzoate, sodium salicylate, sodium acetate, urea, urethane, monoethylacetamide, butazolidine, propylene glycol, the Tween series, amide nicotinate, hexamine, and dimethylacetamide; buffers such as weak acids and salts thereof (acetic acid and sodium acetate), weak bases and salts thereof (ammonia and ammonium acetate), organic compounds, proteins, albumin, peptone, and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂), and ethylenediamine tetraacetic acid; sulfating agents such as 0.1% sodium bisulfide, sodium formaldehyde sulfoxylate, thiourea, disodium ethylenediaminetetraacetate, and acetone sodium bisulfite; a pain relief agent such as benzyl alcohol, chlorobutanol, procaine hydrochloride, glucose, and calcium gluconate; and suspending agents such as sodium CMC, sodium alginate, Tween 80, and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter + cholesterol, lecithin, lanette wax, glycerol monostearate, Tween or span, imhausen, monolan(propylene glycol monostearate), glycerin, Adeps solidus, buytyrum Tego-G, cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro OSI, OSIX, A, B, C, D, H, L, suppository base IV types AB, B, A, BC, BBG, E, BGF, C, D, 299, suppostal N, Es, Wecoby W, R, S, M, Fs, and tegester triglyceride matter (TG-95, MA, 57) may be used.

Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used.

Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Non-limiting examples of the non-aqueous solvent and the suspension include propylene glycol, polyethylene glycol, a vegetable oil such as olive oil, and an injectable ester such as ethyl oleate.

The pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition of the present invention is determined depending on the type of a drug, which is an active ingredient, along with various related factors such as a disease to be treated, administration route, the age, gender, and body weight of a patient, and the severity of diseases.

Additionally, the present invention provides a method of enhancing immunity, comprising administering a composition comprising the *Prevotella merdae* Immunoactis strain of the present invention or a culture solution thereof to a subject in need thereof.

Additionally, the present invention provides a use of a composition comprising the *Prevotella merdae* Immunoactis strain of the present invention or a culture solution thereof for enhancing immunity.

Additionally, the present invention provides a use of the *Prevotella merdae* Immunoactis strain of the present invention or a culture solution thereof for preparing an immunity-enhancing agent.

Additionally, the present invention provides a method of increasing the anticancer effect, comprising administering a composition comprising the *Prevotella merdae* Immunoactis strain of the present invention or a culture solution thereof to a subject in need thereof.

Additionally, the present invention provides a use of a composition comprising the *Prevotella merdae* Immunoactis strain of the present invention or a culture solution thereof for increasing the anticancer effect.

Additionally, the present invention provides a use of the *Prevotella merdae* Immunoactis strain of the present invention or a culture solution thereof for preparing an agent for increasing the anticancer effect.

Additionally, the present invention provides a method of preventing or treating cancer, comprising administering a composition comprising the *Prevotella merdae* Immunoactis strain of the present invention or a culture solution thereof; and an immune checkpoint inhibitor to a subject in need thereof.

Additionally, the present invention provides a use of a composition comprising the *Prevotella merdae* Immunoactis strain of the present invention or a culture solution thereof; and an immune checkpoint inhibitor for preventing or treating cancer.

Additionally, the present invention provides a use of a composition comprising the *Prevotella merdae* Immunoactis strain of the present invention or a culture solution thereof; and an immune checkpoint inhibitor for preparing a cancer treatment agent.

As used herein, the "subject" refers to a subject in need of treatment of a disease, and more specifically, refers to a mammal such as a human or a non-human primate, a mouse, a rat, a dog, a cat, a horse, and a cow, but is not limited thereto.

As used herein, the "administration" refers to providing a subject with a predetermined composition of the present invention by using an arbitrary appropriate method.

The term "prevention" as used herein means all actions that inhibit or delay the onset of a target disease. The term "treatment" as used herein means all actions that alleviate or beneficially change a target disease and abnormal metabolic symptoms caused thereby via administration of the pharmaceutical composition according to the present invention. The term "alleviation" as used herein means all actions that reduce the degree of parameters related to a target disease, e.g., symptoms via administration of the composition according to the present invention.

Additionally, the present invention provides a food composition for enhancing immunity or increasing anticancer effect, comprising the *Prevotella merdae* Immunoactis strain (accession number: KCTC 14922BP) or a culture solution thereof as an active ingredient, wherein the food composition may be a health functional food composition, but is not limited thereto.

When the *Prevotella merdae* Immunoactis strain or a culture solution thereof of the present invention is used as a food additive, it may be added as is or used in combination with other foods or food ingredients, and may be appropriately used according to conventional methods. The mixed amount of the active ingredient may be suitably determined depending on the purpose of use thereof (for prevention or alleviation). In general, when a food or beverage is prepared, the *Prevotella merdae* Immunoactis strain or a culture solution thereof of the present invention is added in an amount of 15 wt% or less, preferably 10 wt% or less based on the raw materials. However, for long-term intake for the purpose of health and hygiene or for the purpose of health control, the amount may be less than the above-mentioned range, and the vesicles have no problem in terms of stability, so the active ingredient may be used in an amount more than the above-mentioned range.

The type of food is not particularly limited. Examples of food to which the material may be added include meats, sausage, bread, chocolate, candies, snacks, confectioneries, pizza, instant noodles, other noodles, gums, dairy products including ice creams, various soups, beverages, tea, drinks, alcoholic beverages, vitamin complexes, and the like, and include all health functional foods in a typical sense.

The health beverage composition according to the present invention may contain various flavors or natural carbohydrates, and the like as additional ingredients as in a typical beverage. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, it is possible to use a natural sweetener such as thaumatin and stevia extract, a synthetic sweetener such as saccharin and aspartame, and the like. The proportion of the natural carbohydrates is generally about 0.01 to 0.20 g, or about 0.04 to 0.10 g per 100 ml of the composition of the present invention.

In addition to the aforementioned ingredients, the composition of the present invention may contain various nutrients, vitamins, electrolytes, flavors, colorants, pectic acids and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for preparing natural fruit juice, fruit juice drinks, and vegetable drinks. These ingredients may be used either alone or in combinations thereof. The proportion of these additives is not significantly important, but is generally selected within a range of 0.01 to 0.20 part by weight per 100 parts by weight of the composition of the present invention.

In the present invention, the term "health functional food" is synonymous with food for special health use (FoSHU) and refers to a food product processed to exhibit effective biological regulatory functions in addition to nutritional supply, having high medical or therapeutic efficacy. The food may be manufactured in various forms such as tablets, capsules, powders, granules, liquids, or pills to obtain beneficial effects in the prevention or improvement of obesity.

The health functional food of the present invention can be prepared by methods conventionally used in the art, and may be produced by adding ingredients and components commonly used in the industry. Furthermore, unlike general pharmaceuticals, since it is derived from food ingredients, it has the advantage of avoiding side effects that may occur with long-term drug administration, and it may also offer excellent portability.

The present invention may be subject to various modifications and may have a variety of embodiments, and specific embodiments are illustrated in the drawings and described in detail in the detailed description. However, this is not intended to limit the invention to specific forms, and it is to be understood that all modifications, equivalents, and substitutions that fall within the spirit and scope of the invention are encompassed therein. In the description of the present invention, detailed explanations of known technologies related to the invention will be omitted where it is determined that such descriptions would obscure the essence of the invention.

Hereinafter, preferred examples are presented to help understand the present invention. However, the following examples are provided only to help understand the present invention more easily, and the contents of the present invention are not limited by the following examples.

### [Modes of the Invention]

### [Examples]

### Example 1. Isolation and identification of Prevotella merdae Immunoactis strain

*Prevotella merdae* Immunoactis (*P. merdae* Immunoactis) was isolated from a patient who responded to anti-PD-1 treatment. Feces from the patient who responded to anti-PD-1 treatment were diluted in phosphate-buffered saline (PBS) and plated on brain heart infusion (BHI) agar medium (#237500, BD Biosciences) supplemented with 0.5% yeast extract (#212750, BD Biosciences), 0.05% L-cysteine-HCl (#C7880, Sigma-Aldrich), 0.0005% hemin (hemin, ##51280, Sigma-Aldrich), 0.0001% vitamin K1 (#95271, Sigma-Aldrich), 0.01% kanamycin (#HKA01, Rd-tech), and 5% defibrinated sheep blood (#S1876, KisanBio). The culture was incubated at 37 °C for 48 hours in an anaerobic incubator (GasPak 100 system, #260626, BD Biosciences). Colonies were randomly selected, and *P. merdae* Immunoactis was isolated using species-specific polymerase chain reaction (PCR).

For whole-genome analysis of the isolated *P. merdae* Immunoactis, genomic DNA was extracted from the pellet of *P. merdae* Immunoactis and sequenced. Specifically, a library was constructed from the genomic DNA using the TruSeq Nano DNA sample prep kit (Illumina) and sequenced. The quality of the sequencing files was confirmed using FastQC (version 0.11.8), and low-quality sequencing reads were removed using Bbduk (version 38.34). A whole-genome assembly was constructed using Unicycler (version 0.4.9b), and strain identification was performed by confirming and comparing the similarity of the whole genome with those of *Prevotella stercorea* DSM 18206 and *Prevotella merdae* sp. Marseille-P4119 using OrthoANI (version 0.93.1). As a result, *P*. *merdae* Immunoactis showed the highest similarity with *Prevotella stercorea* DSM 18206 (78.40%) and *Prevotella merdae* sp. Marseille-P4119 (97.41%), and it was determined to be the same species as *Prevotella merdae* sp. Marseille-P4119 but a different strain.

In addition, to confirm the 16S rRNA sequence of the isolated *P. merdae* Immunoactis, genome annotation was performed using Prokka on the contigs obtained through whole-genome analysis. As a result of confirming the contig corresponding to the 16S rRNA region (partial), it was confirmed that the 16S rRNA of *P. merdae* Immunoactis was encoded with the nucleotide sequence of SEQ ID NO: 1.

**[Table 1]**

| | Sequence (5' → 3') |
|---|---|
| 16S rRNA (SEQ ID NO: 1) | AAGGATGGGGATGCGTCTGATTAGCTTGTTGGCGGGGTAACGGCCCAC CAAGGCGACGATCAGTAGGGGTTCTGAGAGGAAGGTCCCCCACATTGG AACTGAGACACGGTCCAAACTCCTACGGGAGGCAGCAGTGAGGAATA TTGGTCAATGGACGGAAGTCTGAACCAGCCAAGTAGCGTGCAGGAAG ACGGCCCTATGGGTTGTAAACTGCTTTTATACGGGGATAAAGTGAGCC ACGTGTGGCTTATTGCAGGTACCGTATGAATAAGGACCGGCTAATTCC GTGCCAGCAGCCGCGGTAATACGGAAGGTCCGGGCGTTATCCGGATTT ATTGGGTTTAAAGGGAGCGTAGGCCGTTTGTTAAGCGTGTTGTGAAAT GTCGGGGCTCAACCTGGGCATTGCAGCGCGAACTGGCAGACTTGAGTG CGCGGGAAGTAGGCGGAATTCGTCGTGTAGCGGTGAAATGCTTAGATA TGACGAAGAACTCCGATTGCGAAGGCAGCCTGCTGTAGCGTAACTGA CGCTGAAGCTCGAAAGCGTGGGTATCGAACAGGATTAGATACCCTGGT AGTCCACGCGGTAAACGATGGATGCTCGCTGTTTGCGTCTGACGTAAG CGGCCAAGCGAAAGCGTTAAGCATCCCACCTGGGGAGTACGCCGGCA ACGGTGAAACTCAAAGGAATTGACGGGGGCCCGCACAAGCGGAGGAA CATGTGGTTTAATTCGATGATACGCGAGGAACCTTACCCGGGCTTGAA CTGCAGGCGAACGATTCAGAGATGATGAGGCCCTTCGGGGCGCCTGTG GAGGTGCTGCATGGTTGTCGTCAGCTCGTGCCGTGAGGTGTCGGCTTA AGTGCCATAACGAGCGCAACCCTTGCTCTCAGTTGCCATCGGTTAAAG CCGGGCACTCTGTGAGGACTGCCTCCGCAAGGAGTGAGGAAGGTGGGG ATGACGTCAAATCAGCACGGCCCTTACGTCCGGGGCTACACACGTGTT ACAATGGCATGTACAGAAAGCTG |

### Example 2. Preparation of Prevotella merdae Immunoactis strain analysis sample

*Prevotella merdae* Immunoactis isolated in Example 1 was cultured at 37 °C under anaerobic conditions. To maintain anaerobic conditions, AnaeroPack (#A-06, MGC) was added to the anaerobic incubator (Oxoid). *P*. *merdae* Immunoactis was cultured in sterilized BHI medium (#237500, BD Biosciences) supplemented with 0.5% yeast extract (#212750, BD Biosciences), 0.05% L-cysteine·HCl (#C7880, Merck), 0.005% hemin (hemin, #H9039, Sigma-Aldrich), and 0.0001% vitamin K1 (#95271, Sigma-Aldrich). 2 mL of overnight bacterial culture was inoculated into 8 mL of Roswell Park Memorial Institute (RPMI) 1640 medium (#10-040-CV, Corning), and the bacteria were cultured for two days. The RPMI culture was centrifuged at 6,000 rpm for five minutes, and the supernatant was filtered using a 0.2 µm syringe filter (#17823-K, Sartorius). 1 mL aliquots of the supernatant were stored at -80 °C until use. The *P. merdae* Immunoactis culture was centrifuged at 6,000 rpm for five minutes, the supernatant was discarded, and the pellet was resuspended in PBS. The optical density of the resuspended solution at 600 nm (OD₆₀₀) was adjusted to OD₆₀₀ = 6.

### Example 3. T cell proliferation and activation assay

To confirm the immune-related effects of *P. merdae* Immunoactis, human T cells were treated with the culture supernatant of *P. merdae* Immunoactis. Specifically, human T cells were isolated from human blood samples. First, 40 to 50 mL of blood samples were collected from healthy donors in their 20s with prior consent at the Gwangju Institute of Science and Technology (hereinafter, GIST) in Korea. Thereafter, peripheral blood mononuclear cells (PBMCs) were isolated by density gradient centrifugation using Ficoll Paque Plus (#GE17-1440-02, Merck). Next, CD4⁺ T cells and CD8⁺ T cells were isolated using a CD4⁺ T cell isolation kit (#130-096-533, Miltenyi Biotec) and a CD8⁺ T cell isolation kit (#130-096-495, Miltenyi Biotec), respectively. Isolated T cells were resuspended in RPMI 1640 medium (#10-040-CV, Corning) supplemented with 10% fetal bovine serum (FBS) (#92916754, MP Biomedicals), 1% penicillin-streptomycin (#15140-122, Thermo Fisher Scientific), 1× NEAA (#11140-050, Thermo Fisher Scientific), 200 mM 4-(2-hydroxyethyl)-1-piperazineethanesulfonic acid (HEPES) (#15630-080, Thermo Fisher Scientific), 2 mM sodium pyruvate, 2 mM L-glutamine (#25030-081, Thermo Fisher Scientific), and 1×2-mercaptoethanol (#21985-023, Thermo Fisher Scientific). Next, the isolated T cells were stained with carboxyfluorescein succinimidyl ester (CFSE) (#C34554, Thermo Fisher Scientific) for 30 minutes at 37 °C in a 5% CO₂ atmosphere. 96-well untreated plates (#32096, SPL) were pre-coated with 0.2 mg of α-CD3e antibody (#100340, BioLegend) per well, and cells were incubated overnight at 4 °C. CFSE-stained T cells were plated at 2 × 10⁵ per well into the 96-well pre-coated plates (#32096, SPL) and cultured alone or together with 10% *P. merdae* Immunoactis culture supernatant for 72 hours at 37 °C in a 5% CO₂ atmosphere. After 72 hours of culture, T cells were harvested and washed with a fluorescence-activated cell sorting (FACS) buffer (PBS + 10% FBS + 2 mM EDTA). Stained cells were analyzed using CANTO II (BD Biosciences) and BD FACS Diva software v.8.0.2 (BD Biosciences), and data analysis was performed using FlowJo software (v.10, TreeStar).

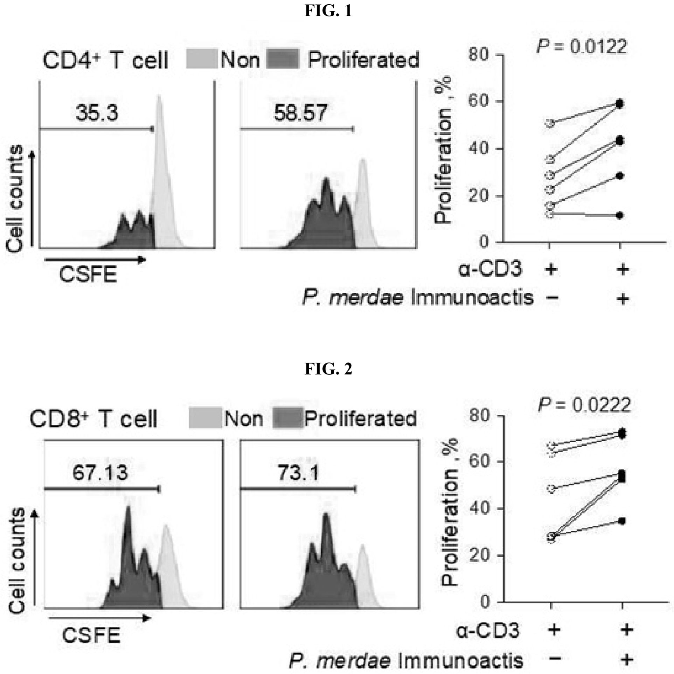

As a result, as shown in FIGS. 1 and 2, combined treatment with the *P. merdae* Immunoactis culture supernatant and α-CD3e antibody significantly promoted the proliferation of CD4⁺ T cells and CD8⁺ T cells compared to when cells were treated with the α-CD3e antibody alone.

In addition, the supernatant of cultured T cells was harvested and stored at -80 °C until used in enzyme-linked immunosorbent assay (ELISA). Thereafter, the interferon-gamma (IFN-γ) level was measured using a human IFN-γ ELISA kit according to the manufacturer's protocol (#88-7316-88, Thermo Fisher Scientific).

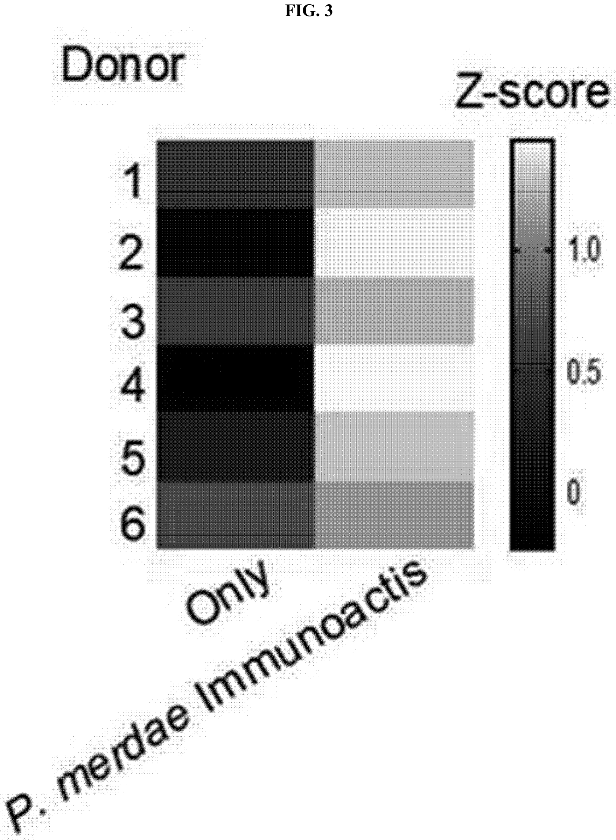

As a result, as shown in FIG. 3, it was confirmed that IFN-γ secretion increased when combined treatment of α-CD3e antibody and *P. merdae* Immunoactis culture supernatant was performed, compared to when cells were treated with the α-CD3e antibody alone.

### Example 4. Synergistic effect of α-PD-1 on tumor suppression by P. merdae Immunoactis administration

To investigate the anticancer effect of *P. merdae* Immunoactis, MC38 colon cancer cells were inoculated into mice, and α-PD-1 was administered to perform syngeneic mouse experiments. Specifically, all animal experiments were conducted according to the protocols approved by the GIST Institutional Animal Care and Use Committee (GIST-2021-096). All animals used in this study were maintained and handled according to the approved policy of the research protocol. MC38 colon cancer cells (#EZH204, Kerafast) were maintained in Dulbecco's Modified Eagle Medium (DMEM) (#11965-118, Thermo Fisher Scientific) supplemented with 10% heat-inactivated FBS (#92916754, MP Biomedicals) and 1% penicillin-streptomycin (Thermo Fisher Scientific) under the conditions of 5% CO₂ and 37 °C. Thereafter, the MC38 cells were passaged at 70% to 80% confluency. A syngeneic tumor model was prepared by subcutaneously injecting 2 × 10⁵ MC38 colon cancer cells into 7-week-old female C57B6/N mice (Orientbio) (day 0). Tumor sizes were measured three times a week until the end of the experiment, and the tumor volume was calculated as length × width² × 0.5. PBS (#10010-049, Thermo Fisher Scientific) and isolated *P. merdae* Immunoactis (OD₆₀₀ = 6, 200 µl) were orally administered six days a week starting 14 days before tumor inoculation (day -14) and continued for three weeks after tumor inoculation (day 23). To investigate the combination therapy, 2 mg/kg of immunoglobulin G (IgG) isotype (clone 2A3, #BE0089, BioXCell) and 2 mg/kg of α-PD-1 mAb (clone RMP1-14, #BE0146, BioXCell) were injected intraperitoneally on days 7, 9, 11, 14, 16, and 18 after tumor inoculation. PBS and IgG isotype were used as controls for bacterial administration and α-PD-1 mAb injection, respectively.

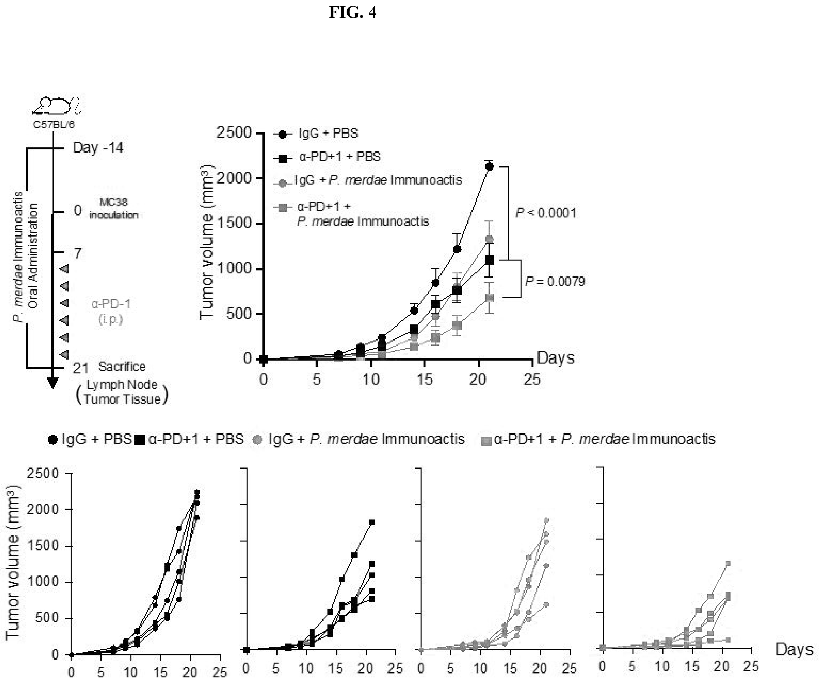

As shown in FIG. 4, the experimental results showed that the combined administration of *P. merdae* Immunoactis and α-PD-1 significantly suppressed tumor growth compared to the administration of α-PD-1 alone.

### Example 5. Enhanced immune activity and penetration into tumor environment of α-PD-1 by administration of P. merdae Immunoactis

To demonstrate the effect of *P. merdae* Immunoactis on the immune system, the expression of immune-related genes and immune cell populations were profiled in tumors of a tumor mouse model administered 2 mg/kg of α-PD-1 and 200 µl of *P. merdae* Immunoactis at OD₆₀₀=6.

Specifically, lymph nodes and tumor tissue were isolated from mice 21 days after MC38 cell inoculation. Thereafter, five immune cell types [T cells, natural killer (NK) cells, dendritic cells (DCs), regulatory T cells (T_{reg} cells), and macrophages] were analyzed. Lymph nodes were ground with 1 mL of PBS (#10010-049, Thermo Fisher Scientific) using a 70 µm cell strainer (#352350, Falcon), and lymphocytes were harvested, washed with PBS, and counted. The tumor tissue was cut into small pieces, transferred to 5 mL of a dissociation medium, and incubated at 37 °C for 40 minutes. The tumor dissociation medium was prepared using a T cell medium supplemented with 2.5 mg/ml collagenase type I (#17100-017, Thermo Fisher Scientific), 1.5 mg/ml collagenase type II (#17101-015, Thermo Fisher Scientific), 1 mg/ml collagenase type IV (#17104-019, Thermo Fisher Scientific), 50 µg/ml DNase type I (#10104159001, Merck), and 0.25 mg/ml hyaluronidase type IV-S (#H3884, Merck). After dissociation, samples were centrifuged, the supernatant was discarded, and cells were resuspended in 5 mL of 1X red blood cell (RBC) lysis buffer (#420301, BioLegend) and incubated at room temperature for 10 minutes. The samples were filtered through a 70 µm cell strainer, and the number of cells was counted. To block Fc receptors, the samples were incubated with anti-mouse CD16/CD32 (Biolegend, #101330) for 10 minutes at room temperature. After Fc blocking, an antibody mixture was added to the samples for surface marker staining and incubated for one hour at 4 °C. For T_{reg} cells and macrophages, the samples were fixed and permeabilized using a fixation/permeabilization buffer set (BioLegend, #424401), and the Ab mixture was added for marker staining and incubated for one hour at 4 °C. The stained cells were analyzed with CANTO II (BD Biosciences) and BDFACS Diva software v. 8.0.2 (BD Biosciences), and data analysis was performed using FlowJo software (v. 10, TreeStar). In addition, the staining markers used for each cell type were as follows:
T cells: CD45 (#103116, BioLegend), CD3 (#100218, BioLegend), CD4 (#100422, BioLegend), CD8a (#563068, BD Biosciences), CD44 (#563970, BD Biosciences), CD62L (#104412, BioLegend), and PD-1 (#109104, BioLegend);
NK cells: CD45 (#103116, BioLegend), CD3 (#100218, BioLegend), NK1.1 (#550627, BD Biosciences), and NKG2A (#11-5896-82, eBioscience);
DC: CD11c (#562782, BD Biosciences), CD11b (#566416, BD Biosciences), and B220 (#103224, BioLegend);
T_{reg} cells: CD45 (#103116, BioLegend), CD3 (#100218, BioLegend), CD4 (#100422, BioLegend), CD8a (#563068, BD Biosciences), CD25 (#101910, BioLegend), Foxp3 (#562996, BD Biosciences), and IFN-g (#163503, BioLegend); and
Macrophages: CD45 (#103116, BioLegend), CD11b (#566416, BD Biosciences), F4/80 (#565411, BioLegend), and TNF-α (#506308, BioLegend).

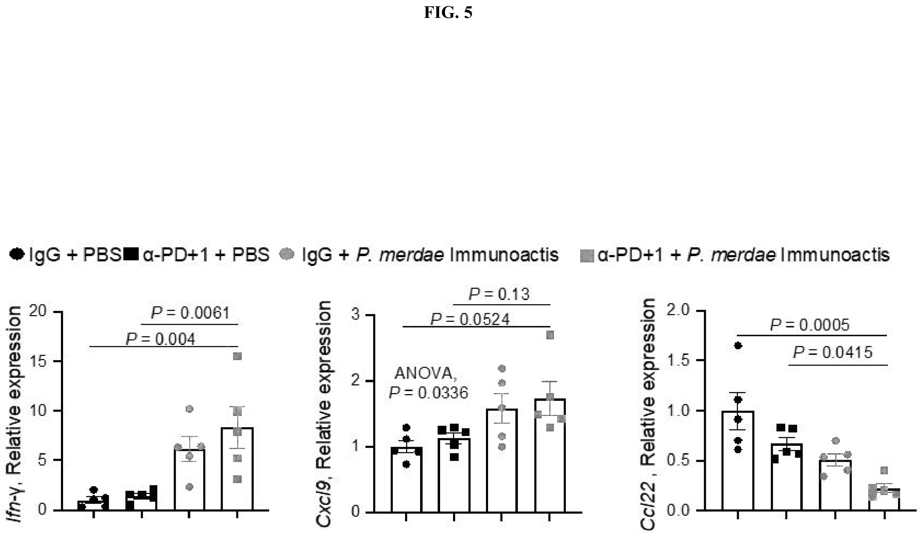

As shown in FIG. 5, the experimental results showed that the expression of *Inf-γ* and *Cxcl9* increased and the expression of *Ccl22* decreased in the tumor tissue of mice co-administered *P. merdae* Immunoactis and α-PD-1 compared to the tumor tissue of mice administered α-PD-1 alone. *Inf-γ* is an important cytokine for innate and adaptive immunity, and *Cxcl9* is one of the chemokines that serve to promote the differentiation and proliferation of leukocytes. In addition, when the expression of *Ccl22* is suppressed, the accumulation of T_{reg} cells may be suppressed, which may result in an antitumor effect.

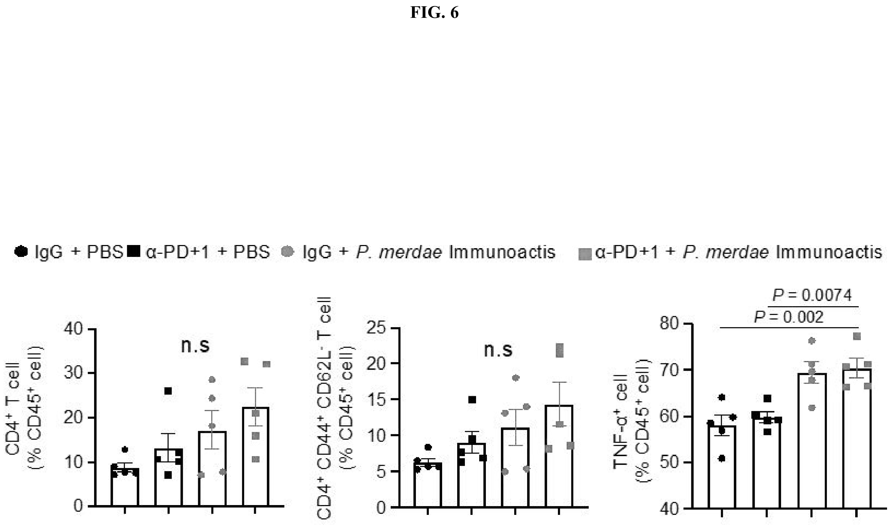

In addition, as shown in FIG. 6, it was confirmed the populations of CD4⁺ T cells, CD4⁺CD44⁺CD62L⁻ T cells (CD4⁺ effector T cells), and TNF-α⁺ cells increased in the tumor tissue of mice administered *P. merdae* Immunoactis and α-PD-1 together compared to the tumor tissue of mice administered α-PD-1 alone.

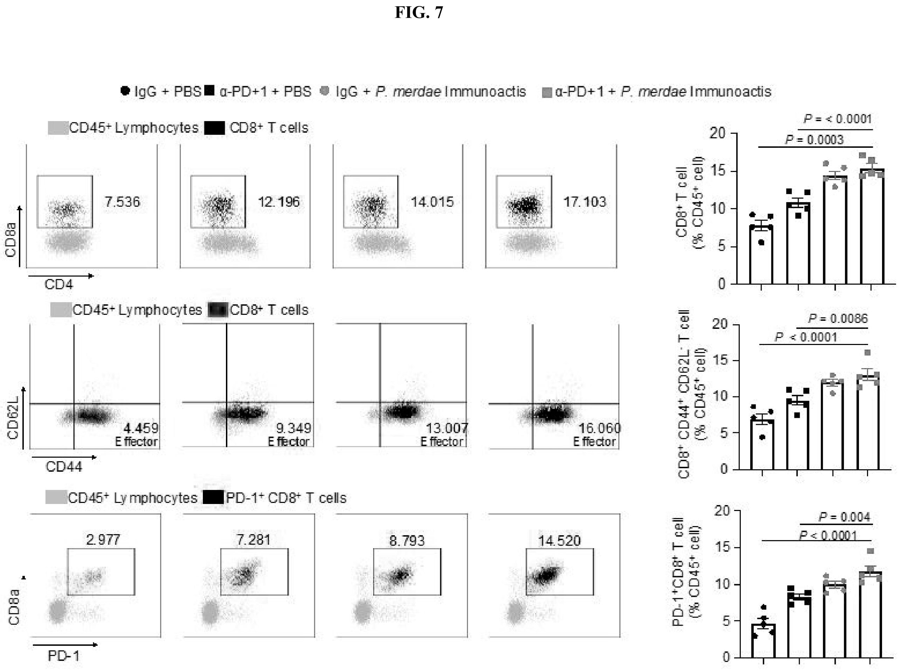

In addition, as shown in FIG. 7, it was confirmed that the populations of CD8⁺ T cells, CD8⁺CD44⁺CD62L⁻ T cells, and PD-1⁺CD8⁺ T cells increased in the tumor tissue of mice administered *P. merdae* Immunoactis and α-PD-1 together compared to the tumor tissue of mice administered α-PD-1 alone.

From the above-described results, it was found that combined administration of α-PD-1 and *P. merdae* Immunoactis improved immune activity and enhanced the activity of penetrating into the tumor environment compared to when PD-1 was administered alone.

### Example 6. Cytotoxicity assay of P. merdae Immunoactis

For cytotoxicity assay, ovalbumin-specific CD8⁺ T cells (OT-1 T cells) were used. Briefly, the spleen was isolated from OT-1 mice (#003831, C57BL/6-Tg[TcraTcrb]1100Mjb/J, The Jackson Laboratory) and ground with 3 mL of PBS using a 70 µm cell strainer (#352350, Falcon). Cells were resuspended in 10 mL of 1X RBC lysis buffer (#420301, BioLegend) and incubated at room temperature for 10 minutes. OT-1 T cells were isolated using a CD8⁺ T Cell Isolation Kit (#130-096-495, Miltenyi Biotec). The isolated OT-1 T cells were resuspended in T cell medium. A 60 mm untreated plate (#32096, SPL) was pre-coated with 5 mg of anti-CD3e antibody (#100340, BioLegend) and 0.5 mg of anti-CD28 antibody (#102116, BioLegend) and incubated overnight at 4 °C. In addition, OT-1 T cells were dispensed into the pre-coated 60 mm plate at 1 to 2×10⁶ cells/well with a 5% bacterial culture supernatant and incubated for 48 hours at 37°C in a 5% CO₂ atmosphere. After 48 hours of incubation, OT-1 T cells were co-cultured with Ova-treated MC38 colon cancer cells at a 5:1 ratio in T cell medium for six hours. Thereafter, cells were harvested and washed with FACS buffer. Finally, cells were stained using an Annexin V-PI staining kit (#ALX-850-020-K101, Enzo Life Science) and analyzed using flow cytometry. Before co-culture with OT-1 T cells, MC38 colon cancer cells were treated with ovalbumin (323-339) (#O1641, Merck) and cultured overnight at 37 °C in a 5% CO₂ atmosphere.

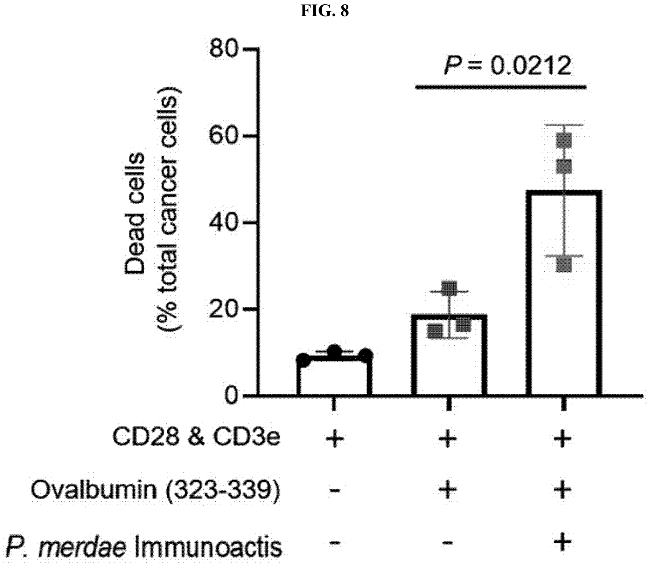

As a result, as shown in FIG. 8, it was confirmed that the ovalbumin-pulsed T cells treated with *P. merdae* Immunoactis culture supernatant killed the MC38 colon cancer cells better. From the above-described results, it was determined that the *P. merdae* Immunoactis strain could increase the cancer cell killing ability of T cells.

The above description of the present invention is for illustrative purposes only, and those skilled in the art will understand that the present invention may be easily modified into other specific forms without changing the technical idea or essential characteristics of the present invention. Therefore, it should be understood that the above-described embodiments are exemplary in all respects and not restrictive.

### [Industrial Applicability]

The novel Prevotella merdae Immunoactis strain (accession number: KCTC 14922BP) of the present invention increases the proliferation of T cells, enhances IFN-γ secretion by T cells, improves the tumor cell-killing ability of T cells, elevates the expression or activity level of IFN-γ or Cxcl9 in the tumor microenvironment, and reduces the expression or activity level of Ccl22. Moreover, it may enhance the anticancer efficacy of immune checkpoint inhibitors. Accordingly, the novel Prevotella merdae Immunoactis strain or a culture solution thereof according to the present invention may be usefully applied for enhancing immunity and increasing the anticancer effect of immune checkpoint inhibitors, and is thus industrially applicable.

### <Accession Number>

Depositary Authority: Korea Research Institute of Bioscience and Biotechnology (KRIBB)
Accession Number: KCTC 14922BP
Date of Deposit: 20220324

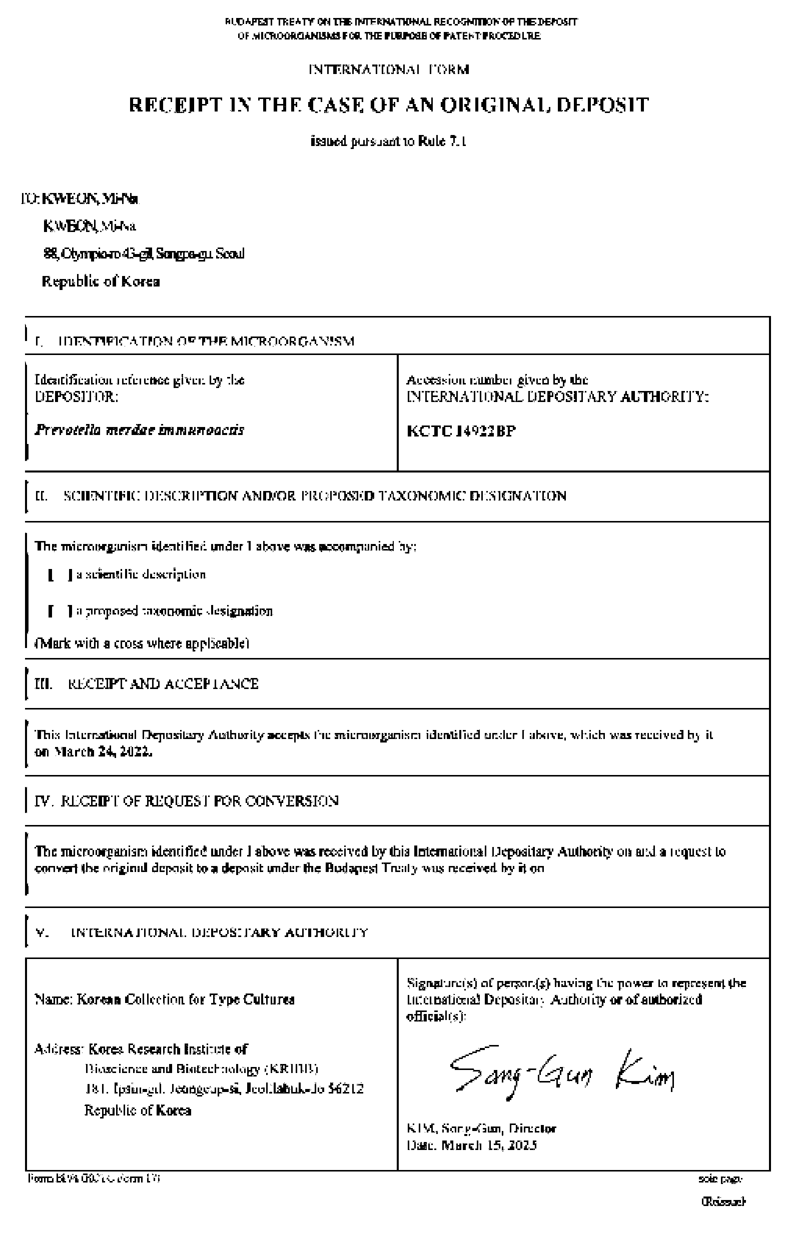
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KWEON, Mi-Na
KWEON, Mi-Na
88, Olympic-ro 43-gil, Songpa-gu Seoul
Republic of Korea

I. IDENTIFICATION OF THE MICROORGANISM
Identification reference given by the
DEPOSITOR:
Prevotella merdae immunoactis | Accession number given by the
INTERNATIONAL DEPOSITARY AUTHORITY:
KCTC 14922BP

II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)

III. RECEIPT AND ACCEPTANCE
This International Depositary Authority accepts the microorganism identified under I above, which was received by it
on March 24, 2022.

IV. RECEIPT OF REQUEST FOR CONVERSION
The microorganism identified under I above was received by this International Depositary Authority on and a request to
convert the original deposit to a deposit under the Budapest Treaty was received by it on

V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: March 15, 2025

Form BP/4 (KCTC Form 17) sole page
(Reissued)

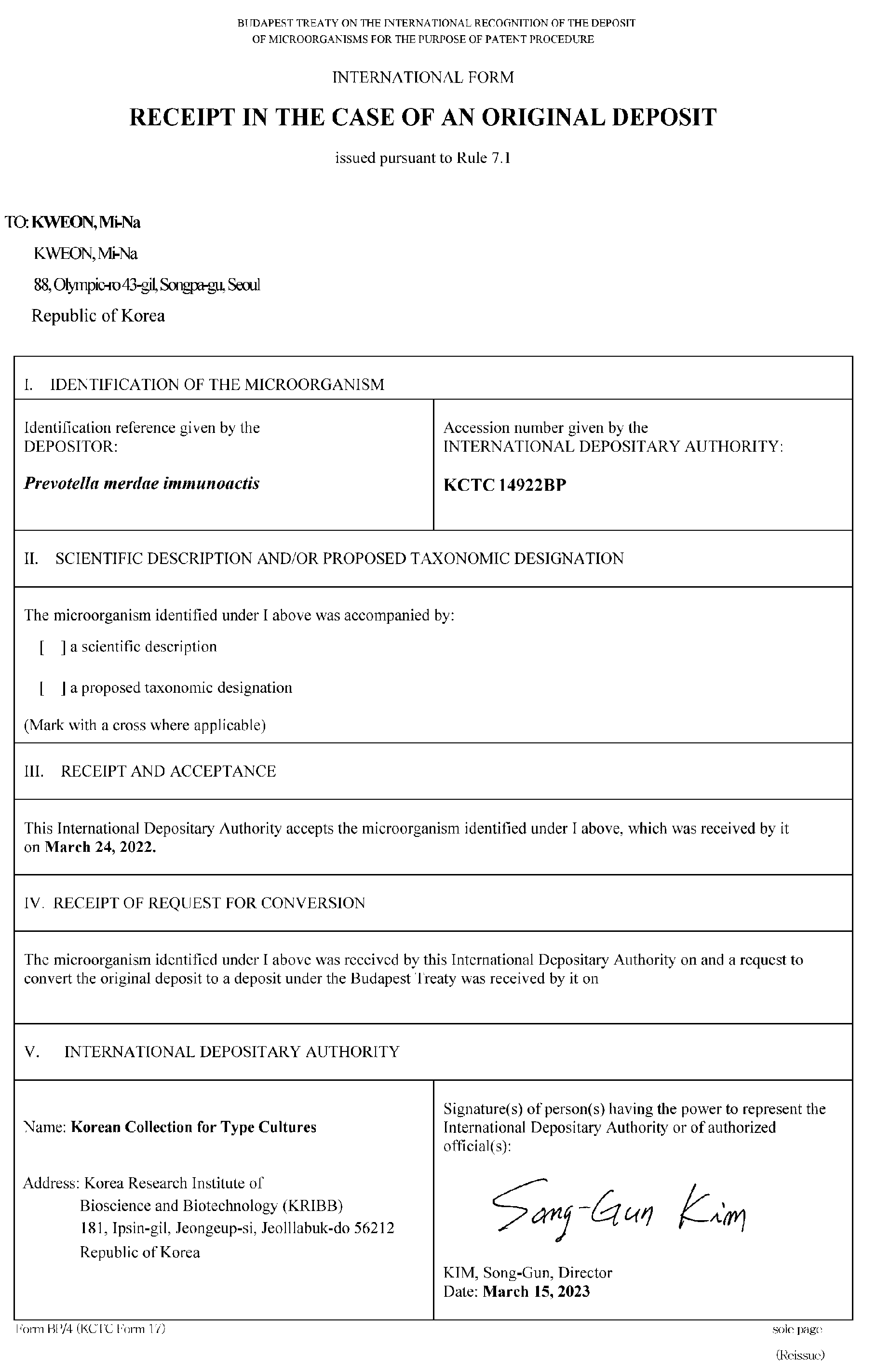
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KWEON, Mi-Na
KWEON, Mi-Na
88, Olympic-ro 43-gil, Songpa-gu, Seoul
Republic of Korea

I. IDENTIFICATION OF THE MICROORGANISM
Identification reference given by the
DEPOSITOR: | Accession number given by the
INTERNATIONAL DEPOSITARY AUTHORITY:
Prevotella merdae immunoactis | KCTC 14922BP

II. SCIENTIFIC DESCRIPTION AND/OR PROPOSED TAXONOMIC DESIGNATION

The microorganism identified under I above was accompanied by:
[ ] a scientific description
[ ] a proposed taxonomic designation
(Mark with a cross where applicable)

III. RECEIPT AND ACCEPTANCE

This International Depositary Authority accepts the microorganism identified under I above, which was received by it
on March 24, 2022.

IV. RECEIPT OF REQUEST FOR CONVERSION

The microorganism identified under I above was received by this International Depositary Authority on and a request to
convert the original deposit to a deposit under the Budapest Treaty was received by it on

V. INTERNATIONAL DEPOSITARY AUTHORITY

Name: Korean Collection for Type Cultures | Signature(s) of person(s) having the power to represent the
International Depositary Authority or of authorized
official(s):
Address: Korea Research Institute of
Bioscience and Biotechnology (KRIBB)
181, Ipsin-gil, Jeongeup-si, Jeolllabuk-do 56212
Republic of Korea |
KIM, Song-Gun, Director
Date: March 15, 2023

Form BP/4 (KCTC Form 17) sole page
(Reissue)

## Claims

1. *A Prevotella merdae* Immunoactis strain deposited under accession number KCTC 14922BP.

2. The strain of claim 1, wherein the strain comprises a DNA sequence encoding a 16S rRNA sequence of SEQ ID NO: 1.

3. The strain of claim 1, wherein the strain enhances immunity or increases an anticancer effect.

4. A composition for enhancing immunity, comprising the *Prevotella merdae* Immunoactis strain of claim 1 or a culture solution thereof as an active ingredient.

5. A pharmaceutical composition for increasing an anticancer effect, comprising the *Prevotella merdae* Immunoactis strain of claim 1 or a culture solution thereof as an active ingredient.

6. The pharmaceutical composition of claim 5, wherein the pharmaceutical composition for increasing an anticancer effect is used in combination with an anticancer drug.

7. A food composition for increasing an anticancer effect, comprising the *Prevotella merdae* Immunoactis strain of claim 1 or a culture solution thereof as an active ingredient.

8. A pharmaceutical composition for preventing or treating cancer, comprising the *Prevotella merdae* Immunoactis strain of claim 1 or a culture solution thereof; and an immune checkpoint inhibitor as active ingredients.

9. The pharmaceutical composition of claim 8, wherein the immune checkpoint inhibitor is a PD-L1 inhibitor or a PD-1 inhibitor.

10. The pharmaceutical composition of claim 9, wherein the PD-L1 inhibitor is one or more selected from the group consisting of atezolizumab, avelumab, durvalumab, envafolimab, cosibelimab, AUNP12, CA-170, and BMS-986189.

11. The pharmaceutical composition of claim 9, wherein the PD-1 inhibitor is one or more selected from the group consisting of nivolumab, pembrolizumab, cemiplimab, spartalizumab, camrelizumab, sintilimab, tislelizumab, toripalimab, dostarlimab, INCMGA00012, AMP-224, and AMP-514.

12. The pharmaceutical composition of claim 8, wherein the *Prevotella merdae* Immunoactis strain or a culture solution thereof; and an immune checkpoint inhibitor are administered simultaneously or sequentially.

13. The pharmaceutical composition of claim 8, wherein the *Prevotella merdae* Immunoactis strain or a culture solution thereof satisfies one or more of the following characteristics:
(a) increasing the proliferation of CD4⁺ T cells and CD8⁺ T cells;
(b) increasing the secretion of interferon-γ (IFN-γ) by T cells;
(c) increasing the expression or activity level of *Inf-γ* or *Cxcl9;* and
(b) decreasing the expression or activity level of *Ccl22.*

14. The pharmaceutical composition of claim 8, wherein the cancer is one or more selected from the group consisting of colorectal cancer, breast cancer, lung cancer, small cell lung cancer, stomach cancer, liver cancer, blood cancer, bone cancer, pancreatic cancer, skin cancer, head or neck cancer, cutaneous or intraocular melanoma, uterine cancer, ovarian cancer, rectal cancer, anal cancer, colon cancer, fallopian tube carcinoma, endometrial carcinoma, cervical cancer, vaginal cancer, vulvar carcinoma, Hodgkin's disease, esophageal cancer, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney cancer, ureteral cancer, renal cell carcinoma, renal pelvic carcinoma, central nervous system tumors, primary central nervous system lymphoma, spinal cord tumors, brainstem glioma, and pituitary adenoma.

15. A method of enhancing immunity, comprising administering a composition comprising the *Prevotella merdae* Immunoactis strain of claim 1 or a culture solution thereof to a subject in need thereof.

16. A use of a composition comprising the *Prevotella merdae* Immunoactis strain of claim 1 or a culture solution thereof for enhancing immunity.

17. A use of the *Prevotella merdae* Immunoactis strain of claim 1 or a culture solution thereof for preparing an immunity-enhancing agent.

18. A method of increasing the anticancer effect of an anticancer drug, comprising administering a composition comprising the *Prevotella merdae* Immunoactis strain of claim 1 or a culture solution thereof to a subject in need thereof.

19. A use of a composition comprising the *Prevotella merdae* Immunoactis strain of claim 1 or a culture solution thereof for increasing the anticancer effect of an anticancer drug.

20. A use of the *Prevotella merdae* Immunoactis strain of claim 1 or a culture solution thereof for preparing an agent for increasing the anticancer effect of an anticancer drug.

21. A method of preventing or treating cancer, comprising administering a composition comprising the *Prevotella merdae* Immunoactis strain of claim 1 or a culture solution thereof; and an immune checkpoint inhibitor to a subject in need thereof.

22. A use of a composition comprising the *Prevotella merdae* Immunoactis strain of claim 1 or a culture solution thereof; and an immune checkpoint inhibitor for preventing or treating cancer.

23. A use of a composition comprising the *Prevotella merdae* Immunoactis strain of claim 1 or a culture solution thereof; and an immune checkpoint inhibitor for preparing a cancer treatment agent.
